# EUROPEAN PATENT SPECIFICATION

(11) **EP 4 217 910 B1**
(45) Date of publication and mention of the grant of the patent: **15.07.2026**
(21) Application number: 21873325.1
(22) Date of filing: 22.09.2021
(51) Int. Cl.: G06K 7/00, G01N 27/327, G01N 33/487

(54) **REAGENT ANALYZER SYSTEM AND METHOD FOR REAGENT STRIP AUTHENTICATION**
REAGENZANALYSESYSTEM UND VERFAHREN ZUR REAGENZSTREIFENAUTHENTIFIZIERUNG
SYSTÈME D'ANALYSE DE RÉACTIF ET PROCÉDÉ D'AUTHENTIFICATION DE BANDE DE RÉACTIF

(30) Priority: 25.09.2020 US 202063083230 P
(43) Date of publication of application: 02.08.2023
(73) Proprietor: Siemens Healthcare Diagnostics Inc., Tarrytown, NY 10591 (US)
(72) Inventor: KAUFFMANN, Aaron, Elkhart, Indiana 46516 (US); DESHPANDE, Manish, Newton, Massachusetts 02461 (US)
(74) Representative: Schweitzer, Klaus
(86) International application number: PCT/US2021/051485
(87) International publication number: WO 2022/066719

(56) References cited:
- EP-A1- 2 341 351
- EP-A2- 0 353 589
- WO-A1-2017/222833
- US-A1- 2010 094 564
- US-A1- 2010 094 564
- US-A1- 2010 140 341
- US-A1- 2014 083 868
- US-A1- 2016 232 363

## Description

This application claims benefit under 35 USC § 119(e) of US Provisional Application No. 63/083,230, filed September 25, 2020.

### BACKGROUND

Early arcade games were easily copied due to lack of encryption. Instead, those early games usually relied on structural protections, such as memory that could be wiped when tampered with. However, these structural protections could be circumvented by various work arounds. Encryption was later adopted and often was only broken by information being lost or leaked.

When downloads and CDs became ubiquitous, encryption/keys were often the default method of protection. These methods of protection were often broken within weeks to months of a product's release-during the highest rate of consumption. Currently, the means of anti-piracy often utilized to ensure long term product protection is a superficial encryption coupled with a deeper protection.

For example, one of the Spyro^{™} games released for the Playstation 1^{™} console was encrypted such that the game file would only run/open on the Playstation^{™} system. Copying the game should have been as simple as burning a new CD with a copy of the original file. There was, however, a specific indexing system included on the original disk. Although, the game could be easily copied to a new CD and played initially, the inclusion of this specific indexing system meant that, as the game was played, slowly, certain parts would stop working, making the game functionally unplayable.

With respect to consumable products, many traditional authentication systems rely on processes that provide immediate detection of non-authentic products, such as the provision of a watermark in bank notes, or holograms in passports. Traditional authentication systems also rely on authentication systems that would impose great difficulty on anyone other than the manufacturer to perform or duplicate, such as processes that require the expense of specialized equipment or those that require a high degree of technical know-how. However, counterfeiters have become increasingly more sophisticated in undermining many traditional authentication systems.

Manufacturers employing an authentication system often do not attempt to hide the fact that their products include an authentication system. Unfortunately, however, these obvious and publicly known authentication systems simply alert possible counterfeiters to the systems that must be subverted to successfully counterfeit a given product. It is, then, usually only a matter of time before counterfeiters are able to undermine publicly known authentication systems. As such, manufacturers face ever increasing costs in implementing new or different authentication systems in an attempt to outpace the work of counterfeiters. This is particularly true among manufacturers of consumables, where it is critical that the end user has confidence that the product is not counterfeit.

In the field of reagent analyzers, for example, where the integrity of consumable products is of the utmost importance, consumable products known as reagent strips are used in the analysis of a variety of substances such as biological fluids and tissues, and commercial or industrial fluids. Reagent strips are affixed with a particular chemical that can indicate the presence of specific particulates within the substances that it is contacted with, when it is analyzed, either manually or instrumentally using a reagent analyzer.

Reagent strips enjoy wide use in many analytical applications, especially in the chemical analysis of biological fluids, because of their relatively low cost, ease of usability, and speed in obtaining results. In medicine, for example, numerous physiological functions can be monitored and/or diagnosed using reagent strips and a reagent analyzer. Such reagent strips provide physicians and laboratory technicians with a facile diagnostic tool.

Because of their widespread use, counterfeit reagent strips have hit the market. However, the performance of counterfeit reagent strips cannot be guaranteed and false readings may be obtained as a result of their use. Such false readings can have drastic implications with respect to the course of treatment a patient may receive.

Currently, manufacturers of reagent strips utilize instantly detectable authentication systems. These instantly detectable authentication systems include a security feature, such as infrared bands on a substrate of the reagent strips. When a particular reagent strip is used and inserted into an analyzer, the security feature is read and a determination is made whether or not the reagent strip is authentic or counterfeit prior to reporting any results from reading a reagent pad on the reagent strip. If the reagent strip is determined to be counterfeit, then the results from reading the reagent pad are not reported to the user. However, such authentication systems can be counterfeit by copying the chemistry and/or IR band. The counterfeit products can also be tested by inserting the counterfeit reagent strip into a reagent analyzer and receiving an immediate determination of whether or not the reagent analyzer detected the counterfeit reagent strip.

EP 0 353 589 A2 discloses apparatus for providing assay calibration data adapted for use in assaying biological samples. Predetermined assay calibration data for normalizing the results of at least one assay with respect to at least one predetermined standard value includes a first code. The first code is used for identifying the at least one assay to which the calibration data corresponds. Apparatus such as a reaction cartridge includes a second code corresponding to the at least one assay. Apparatus responsive to the second code is provided for correlating the second code to the first code to access the calibration data in a storage apparatus.

US 2010/0094564 A1 refers to an analytical strip reading apparatus. The analytical strip comprises at least one reaction zone and at least one optically readable pattern. The optically readable pattern contains identification information of the analytical strip and can be a one-dimensional barcode, a two-dimensional barcode or any optically readable patterns, so as to facilitate reading of the identification information of the analytical strip. The identification information includes a type, a lot number, and an expiration date of the analytical strip as well as reagent calibration data corresponding to the lot number.

Therefore, a need exists for a system and method of making and authenticating a reagent strips that does not alert potential counterfeiters on how to counterfeit it. It is to such consumable products, systems, and methods that the inventive concepts disclosed herein are directed.

### SUMMARY

In some embodiments, the present disclosure describes an authentication system according to independent claim 1, comprising a plurality of reagent strips and an analyzer. Each of the reagent strips has a storage unit and an authentication code stored by the storage unit. The authentication code has a lot value and a secondary value related to the lot value by a predetermined function assigned to the lot value. The analyzer comprises a reader operable to read the authentication code from respective consumables, a controller including a processor, an output device, and a non-transitory computer readable medium storing instructions that, when executed by the processor, cause the processor to: store authentication codes from multiple consumables in the non-transitory computer readable medium; analyze authentication codes from multiple consumables to determine the lot value and the secondary value in the authentication code of each of the consumables; correlate the lot value with the predetermined function; analyze the secondary values associate with a particular one of the lot values to determine whether the secondary values conform with the predetermined function; and perform a predetermined action when the secondary values do not conform with the predetermined function.

By correlating the lot value with the predetermined function and analyzing secondary values obtained from multiple consumables with the predetermined function, a statistical analysis can be used to determine whether or not the secondary values conform with the predetermined function and are authentic consumables. If not, a predetermined action can be taken, such as notifying an operator of counterfeit consumables.

The consumables are reagent strips, and the analyzer is a reagent strip analyzer.

In some embodiments, the present disclosure describes a reagent strip, comprising a substrate and at least one reagent pad positioned on the substrate. The substrate has a storage unit storing an authentication code. The authentication code has a lot value and a secondary value related to the lot value by a predetermined function assigned to the lot value.

In accordance with independent claim 9, a method of authentication is described. In this method, a plurality of respective authentication codes from reagent strips are read with each authentication code having a lot value and a secondary value related to the lot value by a predetermined function assigned to the lot value. The authentication codes are stored in a non-transitory computer readable medium. The authentication codes are analyzed to determine the lot value and the secondary value in the authentication codes. For each lot value, the lot value is correlated with the predetermined function. The secondary values associated with the lot value are analyzed to determine whether the secondary values conform to the predetermined function to determine whether the consumables are authentic. If not, a predetermined action is performed when the secondary values do not conform with the predetermined function such as notifying an operator of a counterfeit consumable.

### BRIEF DESCRIPTION OF THE DRAWINGS

To assist those of ordinary skill in the relevant art in making and using the inventive concepts disclosed herein, reference is made to the appended drawings and schematics, which are not intended to be drawn to scale, and in which like reference numerals are intended to refer to the same or similar elements for consistency. For purposes of clarity, not every component may be labeled in every drawing. Certain features and certain views of the figures may be shown exaggerated and not to scale or in schematic in the interest of clarity and conciseness. In the drawings:
FIG. 1 is a perspective view of an exemplary embodiment of an authentication system according to the inventive concepts disclosed herein.
FIG. 2 is a diagrammatic view of an exemplary embodiment of the authentication system of FIG. 1.
FIG. 3 is a front, plan view of a consumable having an authentication code stored on a storage unit in accordance with the inventive concepts disclosed herein.
FIG. 4 is a front, plan view of a plurality of consumables in a set of consumables having an authentication code stored on a storage unit in accordance with the inventive concepts disclosed herein.
FIG 5a is a diagrammatic view of a reader of the authentication system of FIG. 1 illustrating an optical emission from a PLP spot of the reagent strip in reaction to being excited by the optical emission from the illumination source.
FIG 5b is a diagrammatic view of a reader of the authentication system of FIG. 1 illustrating the reading of an authentication code of the reagent strip.
FIG. 6 is a diagram showing the steps of an exemplary embodiment of a consumable authentication method according to the inventive concepts disclosed herein.

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. The inventive concepts disclosed herein are capable of other embodiments or of being practiced or carried out in various ways. Also, it is to be understood that the phraseology and terminology employed herein is for the purpose of description and should not be regarded as limiting the inventive concepts disclosed and claimed herein in any way.

In the following detailed description of embodiments of the inventive concepts, numerous specific details are set forth in order to provide a more thorough understanding of the inventive concepts. However, it will be apparent to one of ordinary skill in the art that the inventive concepts disclosed herein may be practiced without these specific details. In other instances, well-known features have not been described in detail to avoid unnecessarily complicating the instant disclosure.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherently present therein.

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the inventive concepts. This description should be read to include one or at least one and the singular also includes the plural unless it is obvious that it is meant otherwise.

As used herein, all numerical values or ranges include fractions of the values and integers within such ranges and fractions of the integers within such ranges unless the context clearly indicates otherwise. Thus, to illustrate, reference to a numerical range, such as 1-10 includes 1, 2, 3, 4, 5, 6, 7, 8, 9, 10, as well as 1.1, 1.2, 1.3, 1.4, 1.5, etc., and so forth. Reference to a range of 1-50 therefore includes 1, 2, 3, 4, 5, 6, 7, 8, 9, 10, 11, 12, 13, 14, 15, 16, 17, 18, 19, 20, etc., up to and including 50, as well as 1.1, 1.2, 1.3, 1.4, 1.5, etc., 2.1, 2.2, 2.3, 2.4, 2.5, etc., and so forth. Reference to a series of ranges includes ranges which combine the values of the boundaries of different ranges within the series. Thus, to illustrate reference to a series of ranges, for example, of 1-10, 10-20, 20-30, 30-40, 40-50, 50-60, 60-75, 75-100, 100-150, 150-200, 200-250, 250-300, 300-400, 400-500, 500-750, 750-1,000, includes ranges of 1-20, 10-50, 50-100, 100-500, and 500-1,000, for example.

Further, as used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Finally, as used herein qualifiers such as "about," "approximately," and "substantially" are intended to signify that the item being qualified is not limited to the exact value specified, but includes some slight variations or deviations therefrom, caused by measuring error, manufacturing tolerances, stress exerted on various parts, wear and tear, and combinations thereof, for example.

As discussed above, there exists a need for a system and method for identifying and detecting unauthentic reagent strips in a manner that does not alert potential counterfeiters to an instantly detectable authentication system. The prior art addressed the detection of counterfeit consumable products, but not in a manner that would allow an authentication system to go unnoticed by potential counterfeiters for a period of time after one of the counterfeit consumable products had been used, read by an analyzer, and results reported. The present disclosure describes an authentication system that authenticates reagent strips on a lot by lot basis. In some embodiments, the present disclosure addresses these deficiencies with a system and methodology for authentication of consumables utilizing a two-variable authentication code, wherein the determination of authenticity is not made until after at least one of the counterfeit consumable products has been used without reporting the counterfeit nature of the consumable product.

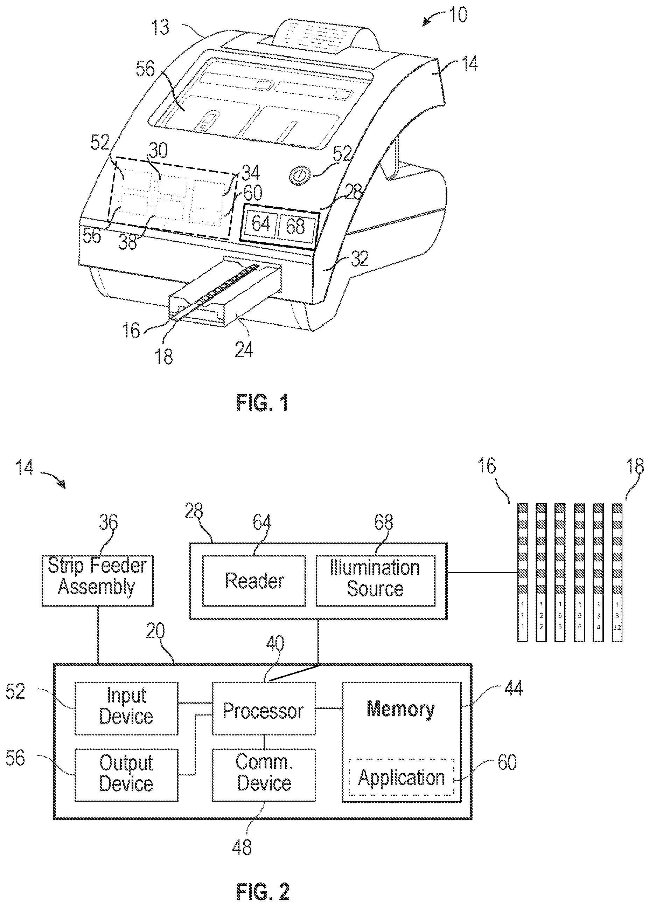

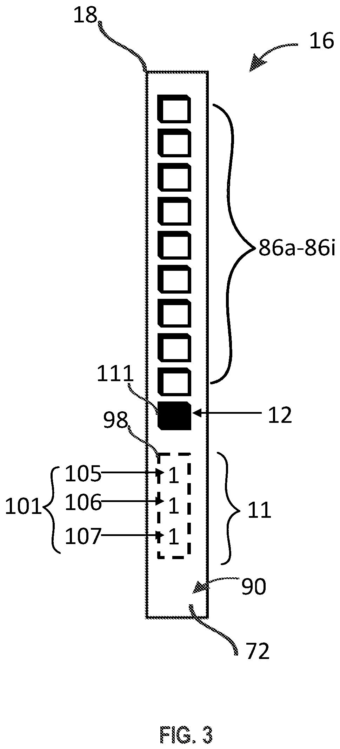

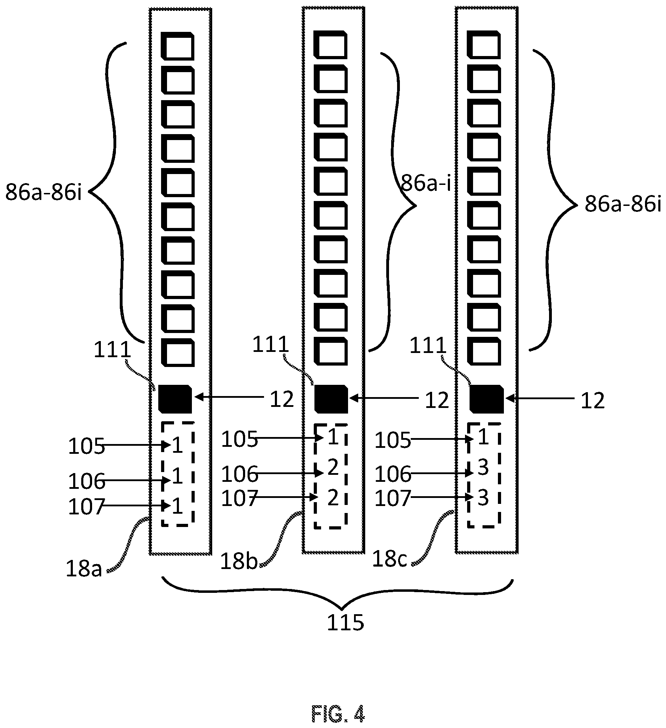

Referring now to FIGS. 1 and 2, shown therein is a perspective view of an exemplary embodiment of an authentication system 10 according to the inventive concepts disclosed herein and a diagrammatic view of the same, respectively. The authentication system 10 may be configured to have a protection scheme 11 (shown in FIGS. 3 and 4) which determines authenticity of a reagent strip on a lot by lot basis, that allows pirates to believe they have been successful in counterfeiting the reagent strip, and a second protection scheme 12 (shown in FIGS. 3 and 4) outside the scope of the appended claims that determines whether or not the reagent strip is authentic or counterfeit prior to reporting any results from reading a reagent pad on the reagent strip. If the reagent strip is determined to be counterfeit, then the results from reading the reagent pad are not reported to the user. By using the protection scheme, the counterfeiter may be initially led to believe that they have successfully counterfeited the reagent strip. But a later determination that indicates that the counterfeiter has not successfully counterfeited the reagent strip may discourage further counterfeiting efforts.

As shown in FIG. 1, The authentication system 10 includes an analyzer 13.
The analyzer 13 is a reagent strip analyzer 14.

The authentication system 10 includes a plurality of consumables 16, as shown in FIG. 2, although only one is shown in FIG. 1. The consumable 16 is a reagent strip 18. The plurality of reagent strips 18 of the authentication system 10 will be discussed in further detail below with regards to FIGS. 3 and 4.

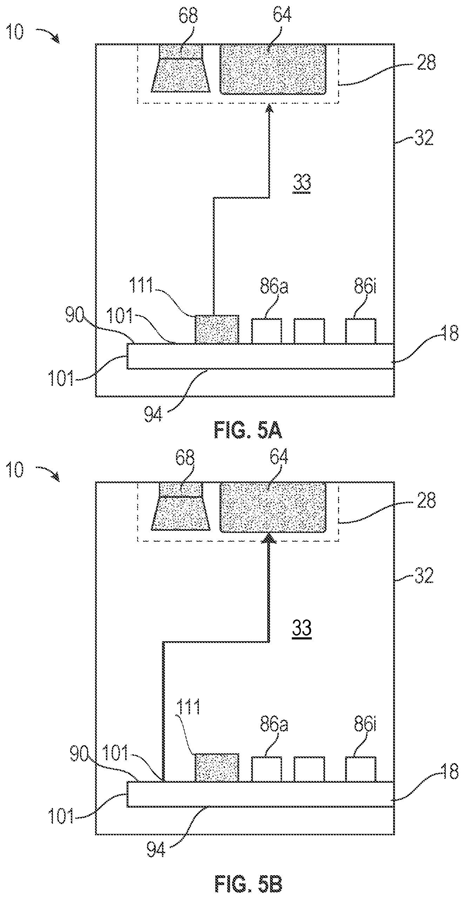

Generally, the reagent strip analyzer 14 includes a controller 20, and may further include a strip tray 24 configured to hold one or more reagent strip 18, and a reading system 28 with a housing 32. The housing 32 encloses a reading space 33 (see FIG. 5A and 5B) sized and configured to receive the reagent strip 18. The housing 32 may be constructed of an opaque material to prevent light from outside the housing 32 from interfering with light being generated in the reading space 33 as discussed below. The housing 32 may be constructed from two or more optically opaque component(s) joined together so as to form and enclose the reading space 33. The reagent strip analyzer 14 may also include an optional waste ramp assembly and a waste receptacle (not shown), for example for disposing of the reagent strip 18 after the reagent strip 18 has been read by the reading system 28. The housing 32 may also be implemented to house and protect the various components of the reagent strip analyzer 14, and to protect technicians and laboratory work surfaces from contamination, for example. The reagent strip analyzer 14 may be provided with a strip feeder assembly 36 for moving the one or more reagent strip 18 from the strip tray 24 into the reading space 33 of the housing 32, past the reading system 28 to the waste receptacle. The strip feeder assembly 36 may include a conveyor, a set of drive wheels or the like. In some embodiments, the reagent strip analyzer 14 may not include a strip tray 24 and/or strip feeder assembly 36, it may, instead, include other features based on implementation. A specific implementation of the strip tray 24 and strip feeder assembly 36 is shown in FIGS 1 and 2 as 24 and 36, respectively.

The controller 20 further includes a processor 40 operably coupled with a non-transitory computer readable medium 44, and may further include a communication device 48, an input device 52, and an output device 56. The controller 20 may be operably coupled with the strip feeder assembly 36 and the reading system 28, for example.

The processor 40 may be implemented as a single processor or multiple processors working together or independently to execute processor executable code, such as application 60, implementing the logic described herein to authenticate the plurality of reagent strips 18, as will be described below. Embodiments of the processor 40 may include a digital signal processor (DSP), a central processing unit (CPU), a microprocessor, a multicore processor, an application specific integrated circuit, a field programmable gate array, and combinations thereof, for example. The processor 40 may be at a location where the plurality of reagent strips 18 are being analyzed by the reagent strip analyzer 14, remotely, or combinations thereof. For example, the processor 40 may be contained within the housing 32, or remotely in a cloud-based computation service. In another embodiment, the processor 40 may include a first processor within the housing 32 and controlling the reading system 28 to obtain the readings from the plurality of reagent strips 18, and a second processor (not shown) receiving and analyzing the readings to determine the authenticity of the plurality of reagent strips 18.

The non-transitory computer readable medium 44 can be implemented as RAM, ROM, flash memory or the like, and may take the form of a magnetic device, optical device, or any other device configured to store processor executable instructions and information in a non-transitory manner, for example. The non-transitory computer readable medium 44 can be a single non-transitory computer readable memory, or multiple non-transitory computer readable memories functioning logically together or independently, for example. The application 60 can be stored in the non-transitory computer readable medium 44, read by the processor 40, and executed by the processor 40 to perform the logic described herein to authenticate the plurality of reagent strips 18 as will be described below.

The input device 52 may be capable of receiving information input from a user and/or processor 40, and transmitting such information to other components of the reagent strip analyzer 14. Implementations of the input device 52 may include, but are not limited to, a keyboard, touchscreen, mouse, trackball, microphone, fingerprint reader, infrared port, slide-out keyboard, flip-out keyboard, cell phone, PDA, remote control, fax machine, wearable communication device, network interface, combinations thereof, and/or the like, for example.

The output device 56 may be capable of outputting information in a form perceivable by the user and/or processor 40. For example, implementations of the output device 56 may include, but are not limited to, a computer monitor, a screen, a touchscreen, a speaker, a website, a television set, a smart phone, a PDA, a cell phone, a fax machine, a printer, a laptop computer, combinations thereof, and the like, for example. It is to be understood that in some exemplary embodiments, the input device 52 and the output device 56 may be implemented as a single device, such as, for example, a touchscreen. It is to be further understood that as used herein the term user is not limited to a human being, and may comprise a computer, a server, a website, a processor, a network interface, a human, a user terminal, a virtual computer, combinations thereof, and/or the like, for example.

The strip tray 24 may be configured to accept the reagent strip 18, for example, and advance the reagent strip 18 so that the reagent strip 18 may be positioned in the reading space 33, and in a field of view of the reading system 28. While the strip tray 24 is illustrated as a retractable mechanism, it should be noted that the strip tray 24 may be implemented as a conveyor belt, a ratchet mechanism, a sliding ramp, or a strip-gripping or pulling mechanism, for example, configured to work with the strip feeder assembly 36 to advance the reagent strip 18 into the field of view of the reading system 28. In some exemplary embodiments, the strip tray 24 and/or the strip feeder assembly 36 may be operably coupled with the controller 20 and configured to move the reagent strip 18 (e.g., in an intermittent and stepwise manner) with a predetermined speed and time between each move so that each reagent strip 18 can be positioned across the field of view of the reading system 28 at known intervals of time, for example.

The reading system 28 may include a reader 64 and an illumination source 68, which may be fixed relative to the strip tray 24 and/or the strip feeder assembly 36, for example. In one embodiment, the reader 64 and the illumination source 68 are mounted to an inside surface of the housing 32.

The reader 64 may be implemented and function as any desired reader, and may be supported at a location above the strip tray 24 or other suitable location, so that a field of view of the reader 64 includes substantially the entire strip tray 24, for example. The reader 64 may be configured to detect, capture, read, image, or otherwise obtain, a signal indicative of the first protection scheme 11 and/or the second protection scheme 12 of the authentication system 10. The reader 64 may include laser scanners, CCD scanners, or imager scanners. The reader 64 may also include any desired digital or analog imager, such as a digital camera, an analog camera, or a CMOS imager, a diode, and combinations thereof, for example. The reader 64 may also include a lens system, optical filters, collimators, diffusers, or any other optical-signal processing device, for example. Further, in some embodiments (not shown), the reading system 28 may include a first reader (not shown) which is an optical imager configured to detect an optical signal in the visible spectrum, and may further include a second reader (not shown) configured to detect an optical signal not in the visible spectrum such as a microwave imaging system, an X-ray imaging system, and other desired imaging systems, for example. The reader 64 may also be configured to transmit a signal indicative of the first protection scheme 11 and/or the second protection scheme 12 to the controller 20, for example. The signal transmitted to the controller 20 by the reader 64 may be an electrical signal, an optical signal, and combinations thereof, for example.

The illumination source 68 may be located adjacent to the reader 64. The illumination source 68 may be implemented as one or more of a light emitting diode, a light bulb, a laser, an incandescent bulb or tube, a fluorescent light bulb or tube, a halogen light bulb or tube, or any other desired light source or object configured to emit an optical signal having any desired intensity, wavelength, frequency, or direction of propagation, for example. The illumination source 68 may be oriented such that substantially the entire field of view of the reader 64 is illuminated by the illumination source 68. In some embodiments, the illumination source 68 may be operably coupled with the controller 20 so that control and/or power signals may be supplied to the illumination source 68 by the controller 20. The optical signals emitted by the illumination source 68 may be conditioned or processed by one or more optical or other systems (not shown), such as filters, diffusers, polarizers, lenses, lens systems, collimators, and combinations thereof, for example. In some embodiments, more than one illumination source 68 may be implemented, such as a first illumination source (not shown) and second illumination source (not shown) having different locations and/or orientations. The first and second illumination sources may emit optical signals having different intensities and/or may be emitted for differing periods of time. In one embodiment, the reading system 28 may be operably coupled with the processor 40 so that one or more power and/or control signals may be transmitted to the reader 64 and/or to the illumination source 68 by the controller 20, and so that one or more signals may be transmitted from the reader 64 to the processor 40, for example.

Referring now to FIGS. 3 and 4, shown therein is a front plan view of a consumable 16, and a plurality of consumables 16 in a set of consumables, respectively, in accordance with the inventive concepts disclosed herein. As discussed above, the consumables, are reagent strips 18.

As shown in FIG. 3, the reagent strip 18 may include a substrate 72, the first protection scheme 11 and the second protection scheme 12 positioned on, or otherwise associated with the substrate 72, and at least one reagent pad 86 positioned on, or otherwise associated with the substrate. Multiple reagent pads 86a-86i are shown by way of example.

The substrate 72 may be constructed of any suitable material, such as paper, photographic paper, polymers, fibrous materials, and combinations thereof, for example. The substrate 72 may have a first side 90 and an opposed second side 94 (see FIG. 5A). The first protection scheme 11, the second protection scheme 12, and the reagent pad 86a-i can be located on the first side 90, the second side 94, or the first protection scheme 11 can be located on one of the first side 90 and the second side 94, and the second protection scheme 12 and/or the reagent pad 86a-i can be located on another one of the first side 90 and the second side 94.

The first protection scheme 11 includes a storage unit 98 operable to store an authentication code 101. The authentication code 101 includes at least a lot value 105 and a secondary value 106. The authentication code 101 further comprises a unique identifier value 107. The authentication code 101, and its constituent lot value 105, secondary value 106, and unique identifier value 107, may be provided as a string of discreet numeric values. The authentication code 101 is stored on the storage unit 98 preferably as a 1D barcode, a 2D barcode, a QR code, and/or as a string of printed numbers. The secondary value 106 is related to the lot value 105 by a predetermined function assigned to the lot value 105. The lot value 105, the secondary value 106, and the unique identifier value 107 may be spaced apart a distance from one another and may be aligned along the longitudinal axis of the reagent strip 18.

The location of the first protection scheme 11 on the substrate 72 may be fixed. In some embodiments, the first protection scheme 11 may be separate from the second protection scheme 12 and/or the reagent pads 86a-i. In some embodiments the first protection scheme 11 may be spatially interposed between the reagent pads 86a-i and the second protection scheme 12. In another embodiment, the first protection scheme 11 may be positioned on the substrate 72 at a first distance from the second protection scheme 12 and a second distance from the reagent pads 86a-i, wherein the second distance is greater than the first distance. In another embodiments, the first protection scheme 11 may be positioned on the substrate 72 at a first distance from the reagent pads 86a-i and a second distance from the second protection scheme 12, wherein the second distance is greater than first distance.

The second protection scheme 12 outside the scope of the appended claims may include, for example, and not by way of limitation, a photo luminescent phosphor (PLP) spot 111. The second protection scheme 12 will be described hereinafter as using the photo luminescent phosphor spot 111, however, it should be understood that the second protection scheme 12 can be implemented in other manners, such as an infrared band having a predetermined and known property. For the purpose of illustration, and not by way of limitation, the second protection scheme 12 will be described as a PLP spot 111. It is to be understood, however, that as used herein the term second protection scheme 12 is not limited to a PLP spot 111, but may also include infrared bands, holograms, radio frequency identification, overt or visible features, combinations thereof, and or the like, for example.

The PLP spot 111 may be formulated such that it would exhibit a specific intensity of luminescence, maximum peak wavelength, rate of decay and kinetics once excited by light. For instance, the PLP spot 111 may be a persistent luminescent phosphor compound such as zinc sulfide (ZnS) doped with copper (ZnS:Cu), cobalt (ZnS:Co), or both copper and cobalt (ZnS:Cu,Co), or strontium aluminate doped with europium and dysprosium (SrAl2O4:Eu2+,Dy3+), for example. These and other persistent luminescent phosphor compounds can be excited by the illumination source 68 and will emit a visible light for a substantial time after the illumination source 68 has been removed. Absorption of light from the illumination source 68 causes excitation of electrons in the compound and produces nonequilibrium concentrations of electrons and holes in the compound. Light is emitted as the electrons recombine with the holes, a process that occurs at a rate dependent on the material that makes up the compound and the temperature (referred to herein as a luminosity decay rate). For instance, copper-doped zinc sulfide produces a yellow-green glow with a maximum wavelength of ~530nm (520nm to 540nm) and the kinetics follow a second order decay rate where 1/intensity is linearly proportional with time. Thus, the resulting emission of visible light may be measured by the reader 64 and four methods of security can be derived from the measured emissions: 1) presence of the compound determined by luminosity (e.g., light is emitted or light having a specific color is emitted), 2) maximum wavelength (each persistent luminescent phosphor compound has a specific maximum wavelength e.g., copper-doped zinc sulfide has a maximum wavelength of ~530nm), 3) luminosity decay rate (each persistent luminescent phosphor compound has a specific luminosity decay rate), and 4) kinetic mechanism (each persistent luminescent phosphor compound has a specific kinetic mechanism e.g., if 1/intensity is linearly proportional with time then the kinetic mechanism is confirmed and the slope of the line is equal to the decay rate constant). While copper-doped zinc sulfide is used as an example, it should be noted that other persistent luminescent phosphor compounds, which have known characteristics as well, may be used to derive the four methods of security from measured emissions.

The PLP spot 111 may be covered or sealed to protect the persistent luminescent phosphor compound from a liquid sample as the liquid sample is being tested. The cover or seal may be formed of any material capable of preventing liquid from contaminating the persistent luminescent phosphor compound while remaining substantially translucent so that light from the illumination source 68 can pass through the cover or seal, and optical emissions from the persistent luminescent phosphor compound can be observed. The location of the PLP spot 111 on the substrate 72 can be fixed. In some embodiments, the PLP spot 111 can be separate from the reagent pads 86a-i and/or the first protection scheme 11. In some embodiments, the PLP spot 111 may be spatially interposed between the first protection scheme 11 and reagent pads 86a-i. In another embodiment, the PLP spot 111 may be positioned on the substrate 72 at a first distance from the first protection scheme 76 and a second distance from the reagent pads 86a-i, wherein the second distance is greater than the first distance. In another embodiments, the PLP spot 111 may be positioned on the substrate 72 at a first distance from the reagent pads 86a-i and a second distance from the first protection scheme 11, wherein the second distance is greater than the first distance.

The reagent pads 86a-i may be arranged in a grid-like configuration on the substrate 72 so as to define the reagent strip 18, for example. In one embodiment, the reagent pads 86a-i are aligned along the longitudinal axis of the reagent strip 18. In an exemplary embodiment, the reagent pads 86a-i may include fluidic or microfluidic compartments (not shown). The reagent pads 86a-i may be spaced apart a distance from one another so that adjacent reagent pads 86a-i may be simultaneously positioned at separate read positions within the field of view of the reading system 28 (FIGS. 1 and 2), for example. The reagent strip 18 may be a multiple-profile reagent strip having multiple reagent pads 86a-i having different reagents. Further, in some exemplary embodiments, the reagent strip 18 may include one or more calibration chip or reference pad, which may have no reagent and may serve as a color reference, for example.

Each reagent pad 86a-i may include a reagent configured to undergo a color change in response to the presence of a target constituent such as a molecule, cell, or substance in a sample of a specimen deposited on the reagent pad 86a-i. The reagent pads 86a-i may be provided with different reagents for detecting the presence of different target constituents. Different reagents may cause one or more color change in response to the presence of a certain constituent in the sample, such as a certain type of analyte. The color developed by a reaction of a particular constituent with a particular reagent may define a characteristic discrete spectrum for absorption and/or reflectance of light for that particular constituent. The extent of color change of the reagent and the sample may depend on the amount of the target constituent present in the sample, for example.

The sample may be any bodily fluid, tissue, or any other chemical or biological sample, and combinations thereof, such as urine, saliva, or blood, for example. The sample may be in liquid form and may contain one or more target constituent such as bilirubin, ketones, glucose, or any other desired target constituent, for example. The presence and concentrations of these target constituent(s) in the sample may be determinable by an analysis of the color change undergone by the one or more reagent pad 86a-i at predetermined times after application of the sample to the reagent pad 86a-i and/or at predetermined read positions in the field of view of the reading system 28, for example. This analysis may involve a color comparison of each reagent pad 86a-i to itself at different time periods after application of the sample and/or at different read positions in the field of view of the reading system 28. In one exemplary embodiment a first read position may be selected as a reference position, and a second read position at which the reagent pad 86a-i is positioned after a predetermined period of time may be selected as a result position, and the test result may be determined from the color change between the reference position and the result position. In this way, the reagent strip 18 may assist in diagnosing the existence of a disease or other health problem by allowing a reagent strip analyzer 14 (FIG. 1-2) to make a qualitative and/or a quantitative or semi-quantitative measurement of a target constituent in a sample.

Now referring to FIG. 4 in combination with FIG. 3, shown in FIG. 4 is a front elevational view of a plurality of reagent strips 18a-c in a set of reagent strips 115 having an authentication code 101 stored on the storage unit 98 (see FIG. 3) in accordance with the inventive concepts disclosed herein. The lot value 105 may be associated with a particular quantity or lot of consumables produced by a manufacturer. As such, the plurality of reagent strips 18a-c from a particular quantity or same manufacturing lot may constitute a set of reagent strips 115, wherein each of the plurality of reagent strips 18a-c in a set of reagent strips 115 share an identical lot value 105, as shown in FIG. 4.

The secondary value 106 is related to the lot value 105 by a predetermined function assigned to the lot value 105. For example, the possible secondary values 106 that may be assigned to the plurality of reagent strips 18a-c in a set of reagent strips 115 may be limited based on the lot value 105 assigned to the said plurality of reagent strips 18a-c. In one embodiment, the secondary values 106 may be assigned based on an algorithm that produces a set of secondary values 106 based on a given lot value 105. For example, the algorithm may produce a set of possible secondary values 106 that is skewed toward a mean dependent on the assigned lot value 105. In another embodiment, the set of possible secondary values 106 may be predetermined by a manufacturer, for example. By way of example, for the plurality of reagent strips 18a-c having a lot value 105 of one (1), the pool of possible secondary values 106 may be 1, 2, and 3. As such, each of the possible secondary values 106 (i.e., 1, 2, and 3) may have a 1/3 chance of being assigned to the plurality of reagent strips 18a-c having a lot value 105 of one (1).

Each of the plurality of reagent strips 18a-c also have a unique identifier value 107 to ensure quality control. The unique identifier value 107 may not be dependent on, or otherwise associated with, either the lot value 105 or the secondary value 106. For example, a unique identifier value 107 may be indicative of a particular one of the pluralities of reagent strips 18a-c within a set of reagent strips 115.

Referring now to FIG. 5a, shown therein is a diagrammatic view of the reader 64 of the authentication system 10 of FIG. 1, illustrating an optical emission from the PLP spot 111 of the reagent strip 18 in reaction to being excited by the optical emission from the illumination source 68.

Outside the scope of the appended claims, the reader 64 may be configured to detect or capture one or more optical or other signals indicative of a luminescence and/or reflectance value of PLP spot 111 and reagent pads 86a-i at any desired read position, and to transmit a signal indicative of the luminescence and/or reflectance value of the PLP spot 111 and/or reagent pads 86a-i at each read position to the processor 40, for example. One or more optical signals having wavelengths indicative of the luminescence and/or reflectance value of the PLP spot 111 and/or reagent pads 86a-i may be detected by the reader 64 at each read position, for example. The signal transmitted to the processor 40 by the reader 64 may be an electrical signal, an optical signal, and combinations thereof, for example. In one embodiment, the signal is in the form of an image file having a matrix of pixels, with each pixel having a color code indicative of its luminescence and/or reflectance value. In an exemplary embodiment, the image file may have two or more predetermined regions of pixels, each predetermined region of pixels corresponding to a read position of one of the PLP spot 111 and/or the reagent pads 86a-i in the field of view of the reader 64.

The processor 40 may determine the color of emissions from PLP spot 111 when activated and/or reflectance value or the color change of reagent pad 86a-i and/or the reagent strip 18 along with a sample (e.g., urine) disposed on the reagent pad 86a-i and/or reagent strip 18, based on signals detected by the reader 64, for example. The color of the PLP spot 111 emissions and/or reflectance value or the color change of reagent pad 86a-i and/or reagent strip 18 may be determined based upon the relative magnitudes of the reflectance signals of various color components, for example, red, green, and blue reflectance component signals. For example, the color of PLP spot 111 emissions may be translated into a standard color model, which typically includes three or four values or color components (e.g., RGB color model, including hue, saturation, and lightness (HLS) and hue, saturation, and value (HSV) representation of points and/or CMYK color model, or any other suitable color model) whose combination represents a particular color. In some embodiments the reader 64 may detect multiple optical signals, with each detected signal having one or more color components, such as a red component signal, a green component signal, and a blue component signal, for example, and each of the component signals may be transmitted to the processor 40 via the same or separate communication link, such as a data bus. In some exemplary embodiments, the reader 64 may detect a single optical signal at each read position, and the processor 40 may translate a signal received from the reader 64 into separate color component signals such as a red component signal, a green component signal, and a blue component signal, for example.

Based upon an analysis of a magnitude of the optical signal detected by the reader 64, the processor 40 may assign the sample to one of a number of categories, e.g., a first category corresponding to no target constituent present in the sample, a second category corresponding to a small concentration of target constituent present in the sample, a third category corresponding to a medium concentration of target constituent present in the sample, and a fourth category corresponding to a large concentration of target constituent present in the sample, for example.

Further, the reader 64 may detect an optical signal indicative of a color or a reflectance value of emissions from the PLP spot 111 at any time interval after excitation by the illumination source 68, and regardless of the particular read position of the PLP spot 111, for example. In one exemplary embodiment, a video, or a sequence of images may be captured of the PLP spot 111 at a variety of time intervals after excitation by the illumination source 68 as the reagent strip 18 is advanced between two or more read positions.

Referring now to FIG. 5b, shown therein is a diagrammatic view of the reader 64 of the authentication system 10 of FIG. 1, illustrating the reading of the authentication code 101 of the reagent strip 18.

The reader 64 is configured to read the authentication code 101 stored on the storage unit 98 of each of the plurality of reagent strips 18a-c, and to transmit a signal indicative of the authentication code 101 to the processor 40, for example. The signal transmitted to the processor 40 by the reader 64 may be an electrical signal, an optical signal, and combinations thereof, for example

Upon receiving a signal from the reader 64, the processor 40 may store the authentication code 101 to the non-transitory computer readable medium 44 of the authentication system 10. The processor 40 then analyzes the authentication codes 101 to determine the lot value 105, secondary value 106, and unique identifier value 107 of the authentication code 101 stored on the storage unit 98 of the plurality of reagent strips 18a-c.

The processor 40 then correlates the lot value 105 with the predetermined function assigned to the lot value 105 before analyzing the secondary value 106 associated with a particular one of the lot values 105 of the plurality of reagent strips 18a-c to determine whether the secondary values 106 conform with the predetermined function assigned to the particular lot value 105 from a particular set of reagent strips 115. The processor 40 then performs a predetermined action if and when the secondary values 106 do not conform with the predetermined function.

In one embodiment, the processor 40 may determine whether the secondary values 106 conform with the predetermined function assigned to the lot value 105 by determining an average assigned value based on an average of the stored secondary values 106 of the authentication codes 101 of each of the plurality of reagent strips 18a-c. In one embodiment, the processor 40 may analyze whether the secondary values 106 associated with a particular one of the lot values 105 conform with the predetermined function assigned to said lot value 105 after storing a predetermined number of authentication codes 101. The predetermined number can be greater than one, and may be in a range from 100 to 1000, 50 to 2000, 25 to 5000 or the like. In another embodiment, the processor 40 may maintain a rolling average of secondary values 106 before determining whether the secondary values 106 conform with the predetermined function assigned to a respective lot value 105.

The predetermined function is the expected mean value based on the lot value 105 assigned to a set of reagent strips 115. The predetermined function may be stored in the non-transitory computer readable memory 44 as a positional look-up data table or in any desired format, for example. Further, the processor 40 may determine whether the secondary values 106 conform with the predetermined function by comparing the secondary values 106 to the predetermined function. The processor 40 may determine that the secondary values 106 conform, or fail to conform, with the predetermined function with a confidence interval between 95%-99.99%, for example. The processor 40 may also perform a predetermined action when the secondary values 106 do not conform with the predetermined function. In one embodiment, the predetermined action may be outputting a notification onto the output device 56 to a user based on the determination that the secondary value 106 fail to conform with the predetermined function.

In one embodiment, the predetermined action may also include outputting a notification to a user based on the determination that a particular authentication code 101, including the unique identifier value 107 assigned to a particular one of the pluralities of reagent strips 18a-c has already been analyzed by the reagent strip analyzer 14.

For example, and not by way of limitation, one or more reagent strip 18 may be assigned secondary values 106 from a group consisting of the numeric values 1, 2, 3, 4, 5, and 6. The expected mean value would be 3.5. If the actual average value was determined to be 3.7 based upon a rolling average of the secondary values 106 on reagent strips 18 analyzed by the analyzer 14, and the predetermined statistical distribution was between 3.0 to 4.0 with a confidence interval of 99.99, then that particular set of reagent strips 115 may not be suspected to be counterfeit.

Another example, and not by way of limitation, if the same aforementioned criteria resulted in a determination of an average value of 4.2, i.e., outside the 99^{th} percentile, that particular set of reagent strips 115 may be suspected of being counterfeit, and the processor 40 may output a notification on to the output device 56 to a user communicating as much, for example.

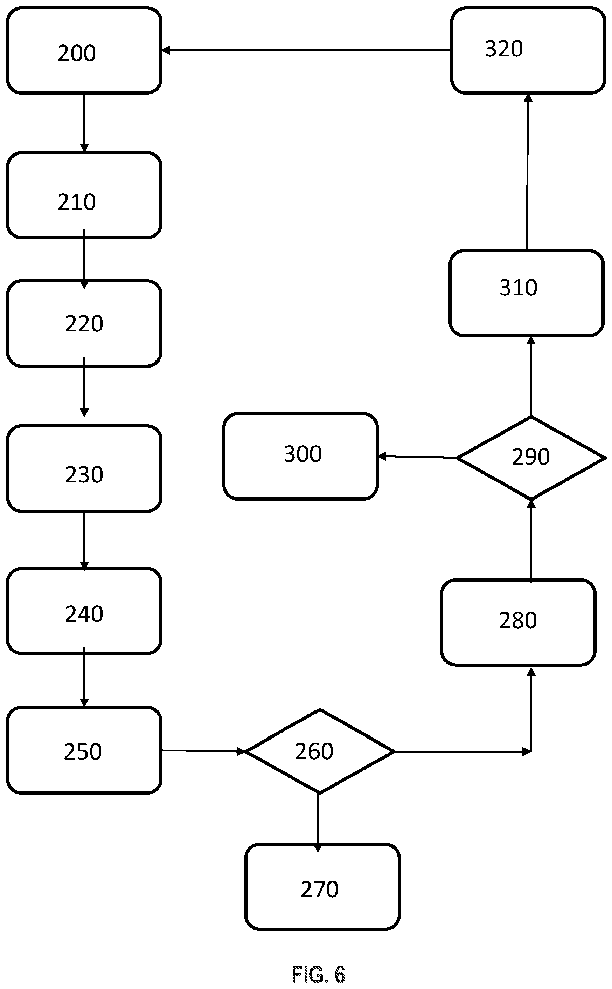

The processor 40 may also inhibit further analysis of a set of reagent strips 115 if the secondary values 106 of a set of reagent strips 115 fails to conform with the predetermined function assigned to the lot value 105 of a set of reagent strips 115, or if the processor 40 determines that the plurality of reagent strips 18a-c have already been analyzed by the reagent strip analyzer 14. This is due to the fact that, generally, reagent strips 18 are vulnerable to counterfeiting. When a counterfeit reagent strip 18 is used, readings from a reagent strip analyzer 14 may be prone to errors, and, thus, false readings may be obtained. Adding at least the first protection scheme 11 having an authentication code 101 security feature to a set of reagent strips 115 that is difficult to reproduce and capable of latent detection ensures that only authentic reagent strips 18 are analyzed. To that end, a consumable authentication method is shown in FIG. 6, which may be carried out by the processor 40 of the authentication system 10. In one embodiment, the authentication routine proceeds as set forth below, as shown in FIG. 6.

The authentication routine will be described below, by way of example, as operating on a single reagent strip 18a. The authentication routine can be repeated to operate on multiple reagent strips 18a-c either sequentially or simultaneously. In other words, the authentication routine can operate on the reagent strip 18a, followed by the reagent strip 18b, etc. Or, the authentication routine can utilize multiple readers 64 to simultaneously operate on multiples of the reagent strips 18a-c.

In step 200 the reagent strip 18 is placed into the field of view of the reader 64 of the authentication system 10 and positioned such that the authentication code 101 stored on the storage unit 98 of the reagent strip 18 is within the field of view of the reader 64. In step 210, the reader 64 reads the authentication code 101 of the reagent strip 18, and in step 220 the reader 64 transmits a signal indicative of the authentication code 101 to the processor 40.

In step 230, the processor 40 stores the authentication code 101 in the non-transitory computer readable medium 44 for further analysis.

In step 240, the processor 40 analyzes the authentication code 101 to determine the lot value 105 and the secondary value 106. In some embodiments, the processor 40 may further determine the unique identifier value 107, if present.

In step 250, the processor 40 correlates the lot value 105 with the predetermined function assigned to the lot value 105. In one embodiment, the predetermined function may be based on the expected mean value of multiple secondary values 106 collected from multiple reagent strips 18.

In step 260, the processor 40 analyzes the secondary value 106 from the reagent strip 18 associated with a particular one of the lot values 105 to determine whether the secondary value 106 from the reagent strip 18, and other reagent strips 18 having the same lot value 105 conform with the predetermined function assigned to said particular lot value 105.

If the processor 40 determines that the secondary values 106 fail to conform to the predetermined function, in step 270 the processor 40 performs a predetermined action. In one embodiment, the predetermined action may include the processor further restricting the reagent strip analyzer 14 from further analyzing the plurality of reagent strips 18a-c. In another embodiment, the predetermined action may include the processor 40 outputting a notification to a user on the output device 56.

If the processor 40 determines that the secondary values 106 conforms with the predetermined function assigned to the lot value 105, in step 280 the processor 40 will analyze the second protection scheme 12.

In step 290, the processor 40 will verify the second protection scheme 12.

If the processor 40 is unable to verify the second protection scheme 12, in step 300, the reagent strip 18 is rejected and the analysis ends for the particular reagent strip 18. The reagent strip 18 can be rejected by not conducting a test of any of the reagent pads 86a-i, or not reporting any test results with respect to any of the reagent pads 86a-i.

If the processor 40 is able to verify the second protection scheme 12, in step 310, the processor 40 will permit the reagent strip analyzer 14 to analyze the reagent pads 86a-i.

In step 320, the processor 40 further analyzes one or more of the reagent strips 18 as described above.

The consumable authentication method may be implemented as a set of processor executable instructions or logic stored on the non-transitory computer readable medium 44, that when executed by the processor 40, cause the processor 40 to carry out the logic to perform the steps as described above.

It is to be understood that the steps disclosed herein may be performed simultaneously or in any desired order. Further, one or more steps may be further divided into one or more sub steps, and two or more steps or sub-steps may be combined in a single step, for example. Further, in some exemplary embodiments, one or more steps may be repeated one or more times, whether such repetition is carried out sequentially or interspersed by other steps or sub-steps. Additionally, one or more step or sub-steps may be carried out before, after, or between, the steps disclosed herein, for example.

From the above description, it is clear that the inventive concepts disclosed herein are well adapted to carry out the objects and to attain the advantages mentioned herein as well as those inherent in the inventive concepts disclosed herein. While exemplary embodiments of the inventive concepts disclosed herein have been described for purposes of this disclosure, it will be understood that numerous changes may be made which will readily suggest themselves to those skilled in the art and which are accomplished within the scope of the inventive concepts disclosed and as defined in the appended claims.

## Claims

1. An authentication system, comprising:
a plurality of consumables (16), each of the consumables having a storage unit (98) and an authentication code (101) stored by the storage unit (98), the authentication code (101) having a lot value (105), a secondary value (106) related to the lot value (105) by a predetermined function assigned to the lot value (105) and a unique identifier value (107),
wherein the plurality of consumables (16) is a plurality of reagent strips (18a-c);
an analyzer (13) comprising:
a reader (64) operable to read the authentication code (101) from respective consumables (16);
a controller (20) including a processor (40), an output device (56), and
a non-transitory computer readable medium (44) storing instructions that, when executed by the processor, cause the processor to:
store authentication codes from multiple consumables (16) in the non-transitory computer readable medium (44);
analyze authentication codes (101) from multiple consumables (16) to determine the lot value (105) and the secondary value (106) in the authentication code of each of the consumables (16);
correlate the lot value (105) with the predetermined function;
analyze the secondary values associated with a particular one of the lot values (105) to determine whether the secondary values (106) conform with the predetermined function; and
perform a predetermined action when the secondary values (106) do not conform with the predetermined function,
wherein the secondary value (106) is assigned to the plurality of reagent strips (18a-c) in a set of reagent strips (115),
wherein the predetermined function is an expected mean value based on the lot value (105) assigned to the set of reagent strips (115).

2. The device of claim 1, wherein the authentication code (101) is stored on the storage unit (98) as one or more of: a 1D barcode, a 2D barcode, a QR code, and a numeric string.

3. The device of any one of claims 1-2, wherein the predetermined function determines an average assigned value based on an average of the secondary values (106).

4. The device of any one of claims 1-3, wherein the predetermined function is a statistical distribution based on an expected assigned value of the secondary values (106).

5. The system of claim 1, wherein the analyzer is a reagent strip analyzer.

6. The device of any one of claims 1-5, wherein the device further comprises an input device, a communication device, or a combination thereof.

7. The device of any one of claims 1-6, wherein the reader (64) is an imager.

8. The device of any one of claims 1-7, wherein the predetermined action causes the processor to output a notification to the output device, restrict further operation of the device, or a combination thereof.

9. A method of authentication, comprising:
reading a plurality of respective authentication codes (101) from consumables (16) each consumable being provided with a storage unit (98) storing the authentication code (101); and
with each authentication code having a lot value (105), a secondary value (106) related to the lot value by a predetermined function assigned to the lot value and a unique identifier value (107);
storing the authentication codes in a non-transitory computer readable medium;
analyzing the authentication codes to determine the lot value and the secondary value in the authentication codes;
for each lot value,
correlating the lot value with the predetermined function;
analyzing the secondary values associated with the lot value to determine whether the secondary values conform to the predetermined function; and
performing a predetermined action when the secondary values do not conform with the predetermined function,
wherein the plurality of consumables (16) is a plurality of reagent strips (18a-c);
wherein the secondary value (106) is assigned to the plurality of reagent strips (18a-c) in a set of reagent strips (115), and wherein the predetermined function is an expected mean value based on the lot value (105) assigned to the set of reagent strips (115).

10. The method of claim 9, wherein the predetermined function
- determines an average assigned value based on an average of the secondary values, and/or
- is a statistical distribution based on an expected assigned value of the secondary values.

11. The method of any one of claims 9-10, further comprising the step of analyzing a reagent pad, and outputting a signal indicative of whether or not a particular analyte of interest is on the reagent pad for at least a group of the reagents strips prior to performing the predetermined action.

## Patentansprüche

1. Authentifizierungssystem, umfassend:
mehrere Verbrauchsmaterialien (16), wobei jedes der Verbrauchsmaterialien eine Speichereinheit (98) und einen Authentifizierungscode (101) aufweist, der durch die Speichereinheit (98) gespeichert ist, wobei der Authentifizierungscode (101) einen Chargenwert (105), einen Sekundärwert (106), der sich auf den Chargenwert (105) bezieht, durch eine vorbestimmte Funktion, die dem Chargenwert (105) zugewiesen ist, und einen eindeutigen Identifizierungswert (107) aufweist,
wobei die mehreren Verbrauchsmaterialien (16) mehrere Reagenzstreifen (18a-c) sind;
einen Analysator (13), umfassend:
ein Lesegerät (64), das betreibbar ist, um den Authentifizierungscode (101) von jeweiligen Verbrauchsmaterialien (16) zu lesen;
eine Steuerung (20), aufweisend einen Prozessor (40), eine Ausgabevorrichtung (56) und
ein nichtflüchtiges computerlesbares Medium (44), das Anweisungen speichert, die, wenn sie durch den Prozessor ausgeführt werden, den Prozessor veranlassen zum:
Speichern von Authentifizierungscodes von mehreren Verbrauchsmaterialien (16) in dem nichtflüchtigen computerlesbaren Medium (44);
Analysieren von Authentifizierungscodes (101) von mehreren Verbrauchsmaterialien (16), um den Chargenwert (105) und den Sekundärwert (106) im Authentifizierungscode jedes der Verbrauchsmaterialien (16) zu bestimmen;
Korrelieren des Chargenwerts (105) mit der vorbestimmten Funktion;
Analysieren der Sekundärwerte, die mit einem bestimmten der Chargenwerte (105) verknüpft sind, um zu bestimmen, ob die Sekundärwerte (106) mit der vorbestimmten Funktion übereinstimmen; und
Durchführen einer vorbestimmten Aktion, wenn die Sekundärwerte (106) nicht mit der vorbestimmten Funktion übereinstimmen, wobei der Sekundärwert (106) den mehreren Reagenzstreifen (18a-c) in einem Satz von Reagenzstreifen (115) zugewiesen ist, wobei die vorbestimmte Funktion ein erwarteter Mittelwert basierend auf dem Chargenwert (105) ist, der dem Satz von Reagenzstreifen (115) zugewiesen ist.

2. Vorrichtung nach Anspruch 1, wobei der Authentifizierungscode (101) auf der Speichereinheit (98) als eines oder mehrere der Folgenden gespeichert ist: ein 1D-Barcode, ein 2D-Barcode, ein QR-Code und eine numerische Zeichenfolge.

3. Vorrichtung nach einem der Ansprüche 1-2, wobei die vorbestimmte Funktion einen mittleren zugewiesenen Wert basierend auf einem Mittelwert der Sekundärwerte bestimmt (106).

4. Vorrichtung nach einem der Ansprüche 1-3, wobei die vorbestimmte Funktion eine statistische Verteilung basierend auf einem erwarteten zugewiesenen Wert der Sekundärwerte ist (106).

5. System nach Anspruch 1, wobei der Analysator ein Reagenzstreifenanalysator ist.

6. Vorrichtung nach einem der Ansprüche 1-5, wobei die Vorrichtung ferner eine Eingabevorrichtung, eine Kommunikationsvorrichtung oder eine Kombination davon umfasst.

7. Vorrichtung nach einem der Ansprüche 1-6, wobei das Lesegerät (64) ein Bildgeber ist.

8. Vorrichtung nach einem der Ansprüche 1-7, wobei die vorbestimmte Aktion den Prozessor veranlasst, eine Benachrichtigung an die Ausgabevorrichtung auszugeben, einen weiteren Betrieb der Vorrichtung einzuschränken, oder eine Kombination davon.

9. Verfahren zur Authentifizierung, umfassend:
Lesen mehrerer jeweiliger Authentifizierungscodes (101) von Verbrauchsmaterialien (16), wobei jedes Verbrauchsmaterial mit einer Speichereinheit (98) versehen ist, die den Authentifizierungscode (101) speichert; und
wobei jeder Authentifizierungscode einen Chargenwert (105), einen Sekundärwert (106), der sich auf den Chargenwert bezieht, durch eine vorbestimmte Funktion, die dem Chargenwert zugewiesen ist, und einen eindeutigen Identifizierungswert (107) aufweist; Speichern der Authentifizierungscodes in einem nichtflüchtigen computerlesbaren Medium;
Analysieren der Authentifizierungscodes, um den Chargenwert und den Sekundärwert in den Authentifizierungscodes zu bestimmen;
für jeden Chargenwert,
Korrelieren des Chargenwerts mit der vorbestimmten Funktion;
Analysieren der Sekundärwerte, die mit dem Chargenwert verknüpft sind, um zu bestimmen, ob die Sekundärwerte mit der vorbestimmten Funktion übereinstimmen; und
Durchführen einer vorbestimmten Aktion, wenn die Sekundärwerte nicht mit der vorbestimmten Funktion übereinstimmen,
wobei die mehreren Verbrauchsmaterialien (16) mehrere Reagenzstreifen (18a-c) sind;
wobei der Sekundärwert (106) den mehreren Reagenzstreifen (18a-c) in einem Satz von Reagenzstreifen (115) zugewiesen ist und wobei die vorbestimmte Funktion ein erwarteter Mittelwert basierend auf dem Chargenwert (105) ist, der dem Satz von Reagenzstreifen (115) zugewiesen ist.

10. Verfahren nach Anspruch 9, wobei die vorbestimmte Funktion
- einen mittleren zugewiesenen Wert basierend auf einem Mittelwert der sekundären Werte bestimmt und/oder
- eine statistische Verteilung ist, die auf einem erwarteten zugewiesenen Wert der Sekundärwerte basiert.

11. Verfahren nach einem der Ansprüche 9-10,
ferner umfassend den Schritt des Analysierens eines Reagenzpads, und Ausgeben eines Signals, das anzeigt, ob sich ein bestimmtes Analyt von Interesse auf dem Reagenzpad befindet oder nicht, für mindestens eine Gruppe der Reagenzstreifen, bevor die vorbestimmte Aktion durchgeführt wird.

## Revendications

1. Système d'authentification, comprenant :
une pluralité de consommables (16), chacun des consommables ayant une unité de stockage (98) et un code d'authentification (101) stocké par l'unité de stockage (98), le code d'authentification (101) ayant une valeur de lot (105), une valeur secondaire (106) liée à la valeur de lot (105) par une fonction prédéterminée attribuée à la valeur de lot (105) et une valeur d'identifiant unique (107),
la pluralité de consommables (16) étant une pluralité de bandes de réactif (18a-c) ;
un analyseur (13), comprenant :
un lecteur (64) utilisable pour lire le code d'authentification (101) à partir de consommables respectifs (16) ;
un dispositif de commande (20) comprenant un processeur (40), un dispositif de sortie (56) et
un support non transitoire lisible par machine (44), stockant des instructions qui, lorsqu'elles sont exécutées par le processeur, amènent le processeur à :
stocker des codes d'authentification provenant de multiples consommables (16) dans le support non transitoire lisible par ordinateur (44) ;
analyser des codes d'authentification (101) à partir de multiples consommables (16) pour déterminer la valeur de lot (105) et la valeur secondaire (106) dans le code d'authentification de chacun des consommables (16) ;
corréler la valeur de lot (105) avec la fonction prédéterminée ; analyser les valeurs secondaires associées à une valeur particulière des lots (105) pour déterminer si les valeurs secondaires (106) sont conformes à la fonction prédéterminée ; et
réaliser une action prédéterminée lorsque les valeurs secondaires (106) ne sont pas conformes à la fonction prédéterminée,
la valeur secondaire (106) étant attribuée à la pluralité de bandes de réactif (18a-c) dans un ensemble de bandes de réactif (115),
la fonction prédéterminée étant une valeur moyenne attendue basée sur la valeur de lot (105) attribuée à l'ensemble de bandes de réactif (115).

2. Dispositif selon la revendication 1, le code d'authentification (101) étant stocké sur l'unité de stockage (98) sous la forme d'un ou plusieurs éléments parmi : un code à barres 1D, un code à barres 2D, un code QR et une chaîne numérique.

3. Dispositif selon l'une quelconque des revendications 1 et 2, la fonction prédéterminée déterminant une valeur attribuée moyenne sur la base d'une moyenne des valeurs secondaires (106).

4. Dispositif selon l'une quelconque des revendications 1 à 3, la fonction prédéterminée étant une distribution statistique basée sur une valeur attribuée attendue des valeurs secondaires (106).

5. Système selon la revendication 1, l'analyseur étant un analyseur de bande.

6. Dispositif selon l'une quelconque des revendications 1 à 5, le dispositif comprenant en outre un dispositif d'entrée, un dispositif de communication, ou une combinaison de ceux-ci.

7. Dispositif selon l'une quelconque des revendications 1 à 6, le lecteur (64) étant un imageur.

8. Dispositif selon l'une quelconque des revendications 1 à 7, l'action prédéterminée amenant le processeur à émettre une notification vers le dispositif de sortie, à restreindre le fonctionnement supplémentaire du dispositif, ou une combinaison de ceux-ci.

9. Procédé d'authentification, comprenant :
la lecture d'une pluralité de codes d'authentification respectifs (101) à partir de consommables (16), chaque consommable étant pourvu d'une unité de stockage (98) stockant le code d'authentification (101) ; et
chaque code d'authentification ayant une valeur de lot (105), une valeur secondaire (106) liée à la valeur de lot par une fonction prédéterminée attribuée à la valeur de lot et une valeur d'identifiant unique (107) ;
le stockage des codes d'authentification dans un support lisible par ordinateur non transitoire ;
l'analyse des codes d'authentification pour déterminer la valeur de lot et la valeur secondaire dans les codes d'authentification ;
pour chaque valeur de lot,
la corrélation de la valeur de lot avec la fonction prédéterminée ;
l'analyse des valeurs secondaires associées à la valeur de lot pour déterminer si les valeurs secondaires sont conformes à la fonction prédéterminée ; et
la réalisation d'une action prédéterminée lorsque les valeurs secondaires ne sont pas conformes à la fonction prédéterminée, la pluralité de consommables (16) étant une pluralité de bandes de réactif (18a-c) ;
la valeur secondaire (106) étant attribuée à la pluralité de bandes de réactif (18a-c) dans un ensemble de bandes de réactif (115), et la fonction prédéterminée étant une valeur moyenne attendue basée sur la valeur de lot (105) attribuée à l'ensemble de bandes de réactif (115).

10. Procédé selon la revendication 9, la fonction prédéterminée
- déterminant une valeur moyenne attribuée sur la base d'une moyenne des valeurs secondaires, et/ou
- étant une distribution statistique basée sur une valeur attribuée attendue des valeurs secondaires.

11. Procédé selon l'une quelconque des revendications 9 à 10, comprenant en outre l'étape d'analyse d'un tampon de réactif, et l'émission en sortie d'un signal indiquant si un analyte particulier d'intérêt se trouve ou non sur le tampon réactif pour au moins un groupe de bandes de réactifs avant d'effectuer l'action prédéterminée.
